# EUROPÄISCHE PATENTSCHRIFT

(11) **EP 0 382 075 B1**
(45) Veröffentlichungstag und Bekanntmachung des Hinweises auf die Patenterteilung: **27.04.1994**
(21) Anmeldenummer: 90101942.2
(22) Anmeldetag: 01.02.1990
(51) Int. Cl.: C07C 403/18, A61K 31/15, A61K 7/48

(54) **Phenylhydrazone des beta-Jonons, ihre Herstellung und daraus hergestellte Arzneimittel und Kosmetika**
Phenyl hydrazones of beta-jonones, their preparation and pharmaceutical and cosmetic compositions prepared therefrom
Phénylhydrazones de bêta-ionone, leur préparation et les compositions pharmaceutiques et cosmétiques préparées à partir de celles-ci

(30) Priorität: 10.02.1989 DE 3903991
(43) Veröffentlichungstag der Anmeldung: 16.08.1990
(73) Patentinhaber: BASF Aktiengesellschaft, 67063 Ludwigshafen (DE)
(72) Erfinder: Janssen, Bernd, Dr., D-6700 Ludwigshafen (DE); Wuest, Hans-Heiner, Dr., 6915 Dossenheim (DE)

(56) Entgegenhaltungen:
- MAKROMOL. CHEM., Band 187, Nr. 7, Seiten 1573-1582; E. DE BARROS LOBO FILHO et al.: "Dégradation contrôlée des polyisoprènes-1,4 par le couple phénylhydrazine/oxygene; Molécules modèles de structures phénylhydrazone conjuguées"
- BERICHTE, Band 66, 1983, Seiten 798-802; R. PUMMERER et al.: "Über den Ozon-Abbau des Carotins und beta-Jonons. (IV. Mitteil. über Carotinoide)"

## Beschreibung

Es ist bekannt, daß Retinsäurederivate (vgl. M.J. Dawson et al., J. Med. Chem. 27, 1516-31 (1984)) und Stilbenderivate [vgl. US 4 326 055, GB 2 164 938 und US 4 588 750], bei welchen die Polyenstruktur der Substanzen des Vitamin-A-Typs in aromatischen Ringen fixiert vorliegt, pharmakologische Wirkungen in experimentellen in-vitro und in-vivo Modellen, bzw. bei der topischen und systemischen Therapie von Neoplasien, Akne, Psoriasis und anderen dermatologischen Affektionen aufweisen. Die Wirkung dieser Verbindungen befriedigt jedoch nicht immer [vgl. G.L. Peck in: The Retinoids, Vol. II, 391-409, Ed.: M.B. Sporn et al., Academic Press N.Y. (1984), oder R.Marks et al., Med. J. Australia 146, 374-377 (1987), oder C.E. Orfanos et al., Drugs 34, 459-503 (1987)].

Der Erfindung lag daher die Aufgabe zugrunde, Verbindungen mit besserem Wirkungsspektrum zu entwickeln.

Überraschend wurde nun gefunden, daß die Phenylhydrazone der Formel I 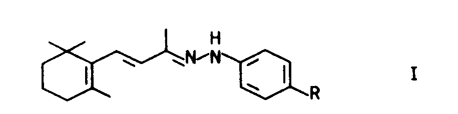
in der R ein Wasserstoffatom, eine Nitro-, C₁-C₄ Alkoxy- oder Alkylgruppe, eine Nitrilgruppe, den Sulfonsäurerest oder die Reste -CONH₂, -CO₂R¹ oder S(O)ₙR² (mit n=0 oder 2), worin R¹ in der Bedeutung von Wasserstoff oder einer C₁-C₃-Alkylgruppe und R² in der Bedeutung einer C₁-C₃-Alkylgruppe steht, bedeutet, sowie deren physiologisch verträgliche Salze ein besseres Wirkspektrum besitzen.

Von den Verbindungen der Formel I sind diejenigen bevorzugt, in denen R für eine -CONH₂-Gruppe, den Sulfonsäurerest oder die Reste -CO₂R¹, -C(O)NR¹OR¹ oder SO₂R² steht, wobei R¹ in der Bedeutung von Wasserstoff und R² in der Bedeutung einer Methyl- oder Ethylgruppe ganz besonders bevorzugt sind.

Die Verbindungen der Formel I, in denen R ein Wasserstoffatom bzw. eine Nitrogruppe bedeutet, sind bereits bekannt und wurden früher üblicherweise hergestellt, um physikalisch leichter charakterisierbare Derivate von Carbonylverbindungen - so auch des β-Jonons - zu erhalten [vgl.: R. Pummerer, L. Rebmann, Ber. 66, 798 + 801 (1933) und E. Labo Filho et al., Makromol. Chem. 187, 1573-82 (1986]. Die hier beschriebene pharmakologische Anwendungsmöglichkeit ist überraschend und neu.

Die Verbindungen der Formel I fallen gegebenenfalls als Gemische von syn-und anti-, bzw. cis- und trans-Isomeren an. Diese lassen sich beispielsweise durch Löslichkeitsunterschiede oder durch allgemein übliche chromatographische Verfahren trennen und in reiner Form isolieren. Sowohl die einheitlichen Isomeren wie auch deren Gemische werden von der vorliegenden Erfindung umfaßt.

Als therapeutische oder kosmetische Mittel kann man sowohl die isomerenreinen Verbindungen wie auch Gemische derselben verwenden.

Einige der erfindungsgemäßen Verbindungen weisen ein acides Wasserstoffatom auf und können daher mit Basen in üblicher Weise in ein physiologisch verträgliches, gut wasserlösliches Salz übergeführt werden. Geeignete Salze sind beispielsweise Ammonium-, Alkalimetall-, insbesondere des Natriums, Kaliums und Lithiums, oder Erdalkalimetallsalze, insbesondere des Calciums oder Magnesiums, sowie Salze mit geeigneten organischen Basen, wie mit niederen Alkylaminen, z.B. Methylamin, Ethylamin oder Cyclohexylamin, oder mit substituierten niederen Alkylaminen, insbesondere hydroxysubstituierten Alkylaminen, wie Diethanolamin, Triethanolamin oder Tris-(hydroxymethyl)-aminomethan sowie mit Piperidin oder Morpholin.

Gegenstand der vorliegenden Erfindung ist ferner ein Verfahren zur Herstellung der oben angegebenen Verbindungen der Formel I, indem man β-Jonon mit Phenylhydrazinen der Formel II 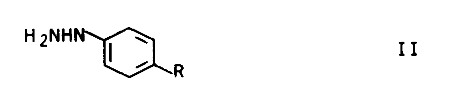
in der R die oben angegebene Bedeutung zukommt, kondensiert. Die Reaktion erfolgt in an sich bekannter Weise [vgl. z.B. "Methoden der Organischen Chemie" Hrsg. Eugen Müller, Bd. VII, 1, S. 461 - 466, Thieme Verlag, Stuttgart 1954 und Bd. VII, 2b, S, 1954 - 1957, Thieme Verlag, Stuttgart 1976 und Bd. X, 2, S. 410 - 414, Thieme Verlag, Stuttgart 1967] gegebenenfalls in Gegenwart eines Lösungs- oder Verdünnungsmittels, gegebenenfalls unter Zusatz eines Katalysators und gegebenenfalls unter Verwendung eines wasserbindenden Mittels bei Temperaturen zwischen 10°C und dem Siedepunkt des Gemisches, wobei man bevorzugt äquimolare Mengen der Reaktanten II und III oder eine Komponente gegebenenfalls im Überschuß von bis zu 15 Mol% umsetzt.

Man kann aber auch β-Jonon in geschützter Form, beispielsweise als offenkettiges oder zyklisches Ketal, unter saurer Katalyse einsetzen.

Zu den bevorzugten Lösungs- und Verdünnungsmitteln gehören Kohlenwasserstoffe, wie Heptan, Cyclohexan, Toluol oder Xylol, ferner niedere aliphatische Alkohole, wie Methanol, Ethanol und Isopropanol, aber auch Cyclohexanol sowie Ethylenglykol, seine Mono- und Dialkylether, Glycerin, ferner Ether wie Diethylether, Diisopropylether und Methyl-tert.-butylether oder Tetrahydrofuran und Dioxan. Desweiteren zu nennen sind Essigsäure, Amide wie Dimethylformamid oder N-Methylpyrrolidon, ferner Pyridin, Sulfolan und Wasser oder entsprechende Gemische.

Als Reaktionsbeschleuniger kommen in Frage Mineralsäuren, wie Salzsäure oder Schwefelsäure, vorzugsweise Carbonsäuren wie Essigsäure sowie deren Alkalisalze. Aber auch Basen wie Pyridin oder Morpholin können als Katalysator dienen.

Als wasserbindende Mittel sind anorganische Salze wie wasserfreies Natriumcarbonat oder Magnesiumsulfat oder auch Molekularsiebe zu verwenden; gegebenenfalls wird beim Arbeiten in lipophilen Medien das gebildete Reaktionswasser ausgekreist.

Die Reaktion wird im allgemeinen drucklos oder unter Druck durchgeführt.

Die erfindungsgemäße Benzoesäure der Formel I (R = CO₂H) kann, falls dies gewünscht ist, in weitere erfindungsgemäße Derivate der Formel I überführt werden, indem man zunächst ihr carbonylaktives Derivat herstellt, z.B. ein Säurehalogenid, -azid, -imidazolid oder -anhydrid, den O-Acyl-N,N′-dicyclohexylharnstoff oder p-Nitrophenylester, und dieses mit Ammoniak zum entsprechenden Amid (R = CONH₂) oder aber mit einem Hydroxylamin der Formel HNR¹OR¹ zum erfindungsgemäßen Hydroxamsäure-Derivat (R = CONR¹OR¹) umsetzt. Die Reaktion erfolgt nach allgemein bekannten Verfahren (vgl. Jerry March, "Advanced Organic Chemistry", Mc Graw-Hill Kogakushu Ltd., Tokyo, 2 Ed. 1977, S. 384 + 385 und dort angegebene Literatur).

Die Herstellung der erfindungsgemäßen Ester der Formel I (R = CO₂R¹) erfolgt bevorzugt analog zu obigem Verfahren durch Alkoholyse des entsprechenden carbonylaktivierten Derivates in an sich bekannter Weise (vgl. l.c.: S. 361-367 und dort angegebene Literatur).

β-Jonon und einige der eingesetzten Phenylhydrazine der Formel II sind Handelsprodukte, bzw. lassen sich letztere herstellen nach allgemein bekannten Methoden zur Synthese aromatischer Hydrazine, beispielsweise durch Reduktion der entsprechenden, aus Anilinderivaten leicht herstellbaren Diazoniumverbindungen (vgl. "Methoden der Organischen Chemie", Hrsg. Eugen Müller, Bd. X, 2, S. 169-315, Thieme Verlag Stuttgart 1967).

Typische Beispiele für erfindungsgemäße Verbindungen sind außer den in den Beispielen genannten Substanzen:
β-Jonon-N-(4-methoxyphenyl)-hydrazon
β-Jonon-N-(4-cyanophenyl)-hydrazon
β-Jonon-N-(4-ethylsulfonylphenyl)-hydrazon
β-Jonon-N-(4-aminocarboxyphenyl)-hydrazon
β-Jonon-N-(4-methylphenyl)-hydrazon
β-Jonon-N-(4-tert.-butoxyphenyl)-hydrazon
β-Jonon-N-(4-ethoxycarbonylphenyl)-hydrazon
β-Jonon-N-(4-phenylsulfonsäure)-hydrazon
β-Jonon-N-(4-isopropylsulfonylphenyl)-hydrazon
β-Jonon-N-(4-hydroxylaminocarbonylphenyl)-hydrazon
Die erfindungsgemäßen Verbindungen und ihre physiologisch verträglichen Salze können aufgrund ihrer pharmakologischen Eigenschaften bei der topischen und systemischen Therapie und auch Prophylaxe von Praekanzerosen und Karzinomen der Haut, der Schleimhäute und inneren Organe sowie bei der topischen und systemischen Therapie von Akne, Psoriasis und anderer mit pathologisch veränderter Verhornung einhergehenden dermatologischen Erkrankungen, insbesondere Ichthyosis, Dariersche Krankheit, Flechte, Leukoplakie, aber auch gegen Ekzeme, Vitiligo, Warzen, Lichtschäden (vorzeitige Alterung) der Haut, ferner gegen trockene Augen und andere Corneopathien sowie zur Behandlung von rheumatischen Erkrankungen, insbesondere solchen entzündlicher oder degenerativer Art, die Gelenke, Muskeln, Sehnen und andere Teile des Bewegungsapparates befallen, verwendet werden. Ein bevorzugtes Indikationsgebiet ist neben der Therapie von dermatologischen Erkrankungen und durch Sonnenlichteinwirkung oder iatrogen, z.B. durch Corticosteroide induzierte Atrophie, bedingten Hautschädigungen die prophylaktische Behandlung von Praekanzerosen und Tumoren.

Die pharmakologischen Wirkungen können beispielsweise in den nachfolgenden Testmodellen aufgezeigt werden: Die erfindungsgemäßen Verbindungen heben an Hamstertrachealgewebe in vitro die nach Vitamin-A-Mangel einsetzende Keratinisierung auf. Die Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel (I) inhibiert wird. Diese Methodik kann aus Cancer Res. 36, 964-972 (1972) oder aus Nature 250, 64-66 (1974) und Nature 253, 47-50 (1975) entnommen werden.

Darüber hinaus werden durch die erfindungsgemäßen Verbindungen die Proliferationsraten bestimmter maligne veränderter Zellen inhibiert. Diese Methodik kann aus J. Natl. Cancer Inst. 60, 1035-1041 (1978), Experimental Cell Research 117, 15-22 (1978) und Proc. Acad. Sci. USA 77, 2937-2940 (1980) entnommen werden.

Die antiarthritische Wirkung der erfindungsgemäßen Verbindungen kann in üblicher Weise im Tierexperiment im Adjuvans-Arthritis- oder Streptokokkenzellwandinduzierten-Arthritis-Modell bestimmt werden. Die dermatologische Aktivität, beispielsweise für die Behandlung von Akne, kann u.a. durch die komedolytische Aktivität und die Fähigkeit nachgewiesen werden, die Anzahl der Zysten im Modell der Rhino-Maus zu reduzieren.

Diese Methode ist von L.H. Kligman et al. in The Journal of Investigative Dermatology 73, 354-358 (1978) beschrieben.

Als weiteres Maß für die dermatologische Aktivität kann die Reduktion der Talgdrüsen und die damit einhergehende verminderte Talgproduktion am Seitenorgan des Hamsters dienen. Diese Methodik ist von E.C. Gomez in J. Am. Dermatol. 6, 746-750 (1982) beschrieben.

Ferner kann die durch die erfindungsgemäßen Verbindungen erzielbare Reversion von Hautschäden, welche durch UV-Licht ausgelöst wurden, in Tiermodellen bestimmt werden. Diese Methodik ist von L.H. Kligman et al. beschrieben in Connect. Tissue Res. 12, 139-150 (1984) und im Journal of the American Academy of Dermatology 15, 779-785 (1986).

Dementsprechend sind ein weiterer Gegenstand der Erfindung therapeutische und kosmetische Mittel zur topischen und systemischen Anwendung, die eine Verbindung der Formel (I) neben üblichen Trägerstoffen oder Verdünnungsmitteln als Wirkstoff enthalten.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen oder kosmetischen Mittel können die erfindungsgemäß zu verwendenden Verbindungen bei lokaler Anwendung in 0,001 bis 1 %iger Konzentration, bevorzugt in 0,001 bis 0,1 %iger Konzentration und bei systemischer Anwendung als therapeutische Mittel vorzugsweise in einer Einzeldosis von 0,1 bis 250 mg enthalten und täglich in einer oder mehreren Dosierungen je nach Art und Schwere der Erkrankungen verabreicht werden.

Die Arzneimittel und Kosmetika der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z.B. Aromastoffe wie Vanillin oder Orangenextrakt, enthalten. Sie können außerdem Suspendierhilfsstoffe wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Zweckmäßige übliche Bestandteile von kosmetischen und pharmazeutischen Zubereitungen für die topische Anwendung sind beispielsweise: anionische, kationische sowie nichtionische Emulgatoren und Emulsionsstabilisatoren, die gleichzeitig Konsistenzgeber oder Gelbildner sein können, wie Polyvinylpyrrolidon, Fettalkohole, Glycerinmonostearat, Polyacrylsäuren, Cellulosederivate und Ethylenoxid-Propylenoxid-Blockpolymere,
feste oder flüssige Ölkomponenten bzw. Fettstoffe mineralischer, pflanzlicher oder tierischer Herkunft, synthetische Esteröle, wie Triglyceridester und Isopropylmyristat,
hydrophile Komponenten, wie Glycerin, Polyethylenglykol und Propylenglykol.

Weiterhin können als Inhaltsstoffe von Kosmetika genannt werden beispielsweise Lichtschutzmittel, Bräunungsmittel, Konservierungsmittel, Antioxidantien, Pigmente, Farbstoffe, ätherische Öle und Parfümöle, Vitamine, Pflanzenextrakte, Collagen usw. Diese Stoffe können beispielsweise dem CTFA, Cosmetic Ingredient Dictionary, 3. Auflage, Washington 1982, entnommen werden.

### Beispiel 1

### β-Jonon-N-(4-carboxyphenyl)-hydrazon

9,6 g (50 mmol) β-Jonon und 7,6 g (50 mmol) Phenylhydrazin-4-carbonsäure wurden in 75 ml Ethanol 0,5 Std. unter Rückfluß gerührt. Nach Erkalten der Reaktionslösung wurde der Niederschlag abfiltriert, mit Ethanol nachgewaschen und getrocknet. Man erhielt 13,0 g der Titelverbindung, Schmp.: 197-202°C.

### Beispiel 2

### β-Jonon-N-(4-methylsulfonylphenyl)-hydrazon

2,5 g (13 mmol) β-Jonon und 2,5 g (13 mmol) 4-Methylsulfonylphenylhydrazin wurden in 40 ml Ethanol 3 Std. unter Rückfluß gerührt. Abdampfen des Lösungsmittels und Umkristallisieren des Rückstandes aus n-Heptan lieferten nach Trocknung 2,1 g der Titelverbindung, Schmp.: 116-118°C.

### Beispiele für pharmazeutische Zubereitungen:

### Beispiel I

### Tablette mit 250 mg Wirkstoff

Zusammensetzung für 1000 Tabletten:

| | |
|---|---|
| Wirkstoff des Beispiels Nr. 2: | 250 g |
| Kartoffelstärke: | 100 g |
| Milchzucker: | 50 g |
| Gelatinelösung 4 %: | 45 g |
| Talkum: | 10 g |

### Herstellung:

Der fein gepulverte Wirkstoff, Kartoffelstärke und Milchzucker werden gemischt. Die Mischung wird mit ca. 45 g 4 % Gelatinelösung durchfeuchtet, feinkörnig granuliert und getrocknet. Das trockene Granulat wird gesiebt, mit 10 g Talkum vermischt und auf einer Rundläufer-Tablettiermaschine zu Tabletten verpreßt. Die Tabletten werden in dicht schließende Behälter aus Polypropylen gefüllt.

### Beispiel II

Creme aus 0,1 % Wirkstoff

| | |
|---|---|
| Wirkstoff des Beispiels Nr. 1: | 0,1 g |
| Glycerinmonostearat: | 10,0 g |
| Cetylalkohol: | 4,0 g |
| Polyethylenglykol-400-stearat: | 10,0 g |
| Polyethylenglykol-sorbitanmonostearat: | 10,0 g |
| Propylenglykol: | 6,0 g |
| p-Hydroxybenzoesäuremethylester: | 0,2 g |
| Entmineralisiertes Wasser: | ad 100,0 g |

### Herstellung:

Der feinst gepulverte Wirkstoff wird mit Propylenglykol suspendiert und die Suspension in die auf 65°C erwärmte Schmelze aus Glycerinmonostearat, Cetylalkohol, Polyethylenglykol-400-stearat und Polyethylenglykolsorbitanmonostearat gerührt. In diese Mischung wird die 70°C heiße Lösung des p-Hydroxybenzoesäuremethylesters in Wasser emulgiert. Nach dem Erkalten wird die Creme über eine Kolloidmühle homogenisiert und in Tuben abgefüllt.

### Beispiel III

Puder mit 0,1 % Wirkstoff

| | |
|---|---|
| Wirkstoff des Beispiels Nr. 2: | 0,1 g |
| Zinkoxid: | 10,0 g |
| Magnesiumoxid: | 10,0 g |
| Hochdisperses Siliciumdioxid: | 2,5 g |
| Magnesiumstearat: | 1,0 g |
| Talkum: | 76,4 g |

### Herstellung

Der Wirkstoff wird auf einer Luftstrahlmühle mikronisiert und mit den anderen Bestandteilen homogen vermischt. Die Mischung wird durch ein Sieb (Maschenweite Nr. 7) geschlagen und in Polyethylenbehälter mit Streueinsatz abgefüllt.

## Patentansprüche

1. β-Jonon-phenylhydrazone der Formel I, 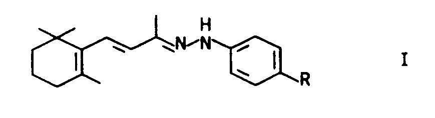 in der R eine C₁-C₄-Alkoxy- oder Alkylgruppe, eine Nitrilgruppe, den Sulfonsäurerest oder die Reste -CONH₂, -CO₂R¹, -C(O)NR¹OR¹ oder S(O)ₙR² (mit n=0 oder 2) bedeutet, worin R¹ in der Bedeutung von Wasserstoff oder einer C₁-C₃-Alkylgruppe und R² in der Bedeutung einer C₁-C₃-Alkylgruppe steht, sowie deren physiologisch verträgliche Salze.

2. Verfahren zur Herstellung der β-Jonon-phenylhydrazone der Formel I gemäß Anspruch 1, dadurch gekennzeichnet, daß man β-Jonon mit Phenylhydrazinen der Formel II 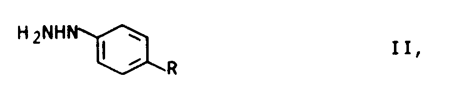 in der R die im Anspruch 1 angegebene Bedeutung hat, umsetzt.

3. Arzneimittel zur topischen Anwendung, das neben üblichen galenischen Hilfsstoffen als Wirkstoff 0,001 bis 1 Gewichtsprozent eines β-Jonon-phenylhydrazons der Formel I gemäß Anspruch 1, oder einer Verbindung der Formel I gemäß Anspruch 1, in der R die Bedeutung von Wasserstoff oder einer Nitrogruppe zukommt, enthält.

4. Arzneimittel zur systemischen Anwendung, das neben üblichen galenischen Hilfsmitteln als Wirkstoff pro Einzeldosis 0,1 bis 250 mg eines β-Jonon-phenylhydrazons der Formel I gemäß Anspruch 1, oder einer Verbindung der Formel I gemäß Anspruch 1, in der R die Bedeutung von Wasserstoff oder einer Nitrogruppe zukommt, enthält.

5. Kosmetische Zubereitung, die neben üblichen kosmetischen Hilfsmitteln 0,001 bis 1 Gew.% eines β-Jonon-phenylhydrazons der Formel I gemäß Anspruch 1, oder einer Verbindung der Formel I gemäß Anspruch 1, in der R die Bedeutung von Wasserstoff oder einer Nitrogruppe zukommt, enthält.

6. Arzneimittel oder Kosmetikum nach Anspruch 3 oder 5 zur Behandlung von Akne oder Psoriasis oder anderen mit pathologisch veränderter Verhornung einhergehenden dermatologischen Erkrankungen sowie von Ekzemen, Warzen und Vitiligo.

7. Arzneimittel oder Kosmetikum nach Anspruch 3 oder 5 zur Behandlung von durch UV-Licht oder iatrogen bedingten Schädigungen der Haut.

8. Arzneimittel nach Anspruch 3 oder 4 zur Behandlung von Präkanzerosen und Tumoren.

9. Arzneimittel nach Anspruch 3 oder 4 zur Behandlung von rheumatischen und arthritischen Erkrankungen.

10. Arzneimittel nach Anspruch 3 oder 4 zur Behandlung von trockenen Augen und anderen Corneopathien.

## Claims

1. A β-ionone phenylhydrazone of the formula I 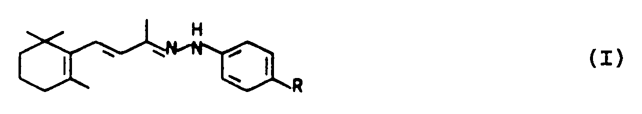 where R is C₁-C₄-alkoxy or -alkyl, cyano, sulfo, or -CONH₂, -CO₂R¹, -C(O)NR¹OR¹ or S(O)ₙR² (with n=0 or 2), where R¹ is hydrogen or C₁-C₃-alkyl, and R² is C₁-C₃-alkyl, and the physiologically tolerated salts thereof.

2. A process for the preparation of a β-ionone phenylhydrazone of the formula I as claimed in claim 1, which comprises reacting β-ionone with a phenylhydrazine of the formula II 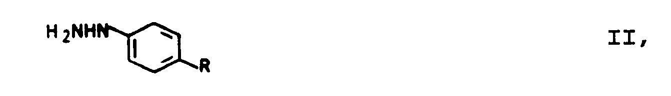 where R has the meaning specified in claim 1.

3. A drug for topical use which contains 0.001 to 1% by weight of a β-ionone phenylhydrazone of the formula I as claimed in claim 1, or of a compound of the formula I as claimed in claim 1 in which R is hydrogen or nitro, as active substance in addition to conventional pharmaceutical auxiliaries.

4. A drug for systemic use which contains 0.1 to 250 mg of a β-ionone phenylhydrazone of the formula I as claimed in claim 1, or of a compound of the formula I as claimed in claim 1 in which R is hydrogen or nitro, as active substance per single dose in addition to conventional pharmaceutical auxiliaries.

5. A cosmetic formulation which contains 0.001 to 1% by weight of a β-ionone phenylhydrazone of the formula I as claimed in claim 1, or of a compound of the formula I as claimed in claim 1 in which R is hydrogen or nitro, in addition to conventional cosmetic auxiliaries.

6. A drug or cosmetic as claimed in claim 3 or 5 for the treatment of acne or psoriasis or other dermatological disorders associated with pathological keratinization, and of eczema, warts and vitiligo.

7. A drug or cosmetic as claimed in claim 3 or 5 for the treatment of UV-induced or iatrogenic damage to the skin.

8. A drug as claimed in claim 3 or 4 for the treatment of precanceroses and tumors.

9. A drug as claimed in claim 3 or 4 for the treatment of rheumatic and arthritic disorders.

10. A drug as claimed in claim 3 or 4 for the treatment of dry eyes and other corneopathies.

## Revendications

1. β-ionon-phénylhydrazones de formule I 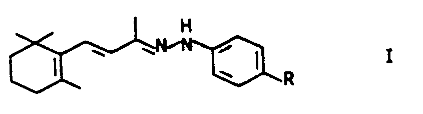 dans laquelle R représente un groupe alcoxy ou alkyle en C1-C4, un groupe nitrile, le reste acide sulfonique et les restes -CONH₂, CO₂R¹, -C(O)NR¹OR¹ ou S(O)ₙR² (avec n = 0 ou 2), où R¹ est mis pour hydrogène ou groupe alkyle en C1-C3 et R² pour groupe alkyle en C1-C3, ainsi que leurs sels acceptables physiologique.

2. Procédé de préparation de β-ionon-phénylhydrazones de formule I selon la revendication 1, caractérisé par le fait que l'on fait réagir du β-ionone avec des phénylhydrazines de formule II 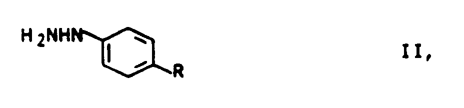 dans laquelle R a la signification donné dans la revendication 1.

3. Médicament pour application topique, qui, outre les additifs galéniques usuels, contient, comme principe actif, 0,001 à 1 % en poids d'une β-ionon-phénylhydrazone de formule I selon la revendication 1 ou un composé de formule I selon la revendication 1 dans laquelle R signifie hydrogène ou groupe nitro.

4. Médicament pour application systémique qui, outre les additifs galéniques usuels, contient, comme principe actif, par dose individuelle de 0,1 à 250 mg, une β-ionon-phénylhydrazone de formule I selon la revendication 1 dans laquelle R signifie hydrogène ou groupe nitro.

5. Préparation cosmétique qui outre les additifs galéniques usuels, contient, comme principe actif, 0,001 à 1 % en poids d'une β-ionon-phénylhydrazone de formule I selon la revendication 1 ou un composé de formule I selon la revendication 1 dans laquelle R signifie hydrogène ou groupe nitro.

6. Médicament ou cosmétique selon la revendication 3 ou 5 pour traiter l'acné ou le psoriasis ou autres maladies dermatologiques aboutissant à des altérations cutanées pathologiques ainsi que les eczémas, verrues et vitiligo.

7. Médicament ou cosmétique selon la revendication 3 ou 5 pour traiter des dommages de la peau produits par la lumière UV ou par introgène.

8. Médicament selon la revendication 3 ou 4 pour traiter des précancéroses et tumeurs.

9. Médicament selon la revendication 3 ou 4 pour traiter des manifestations rhumatismales et arthritiques.

10. Médicament selon la revendication 3 ou 4 pour traiter les yeux secs et autres cornéopathies.
